# EUROPEAN PATENT APPLICATION

(11) **EP 4 790 726 A1**
(43) Date of publication of application: **12.08.2026**
(21) Application number: 25156626.1
(22) Date of filing: 07.02.2025
(51) Int. Cl.: G21F 3/02, G21F 3/03, A61B 6/10

(54) **SHAPEABLE RADIATION-PROTECTIVE DEVICE**

(71) Applicant: Texray AB, 412 92 Göteborg (SE)
(72) Inventor: APELL, Petra, 439 37 ONSALA (SE); GELLERSTEDT, Fredrik, 413 09 GÖTEBORG (SE)
(74) Representative: Öberg & Sjöberg AB

(57) **Abstract**

A wearable radiation-protective device (10) is proposed that has an intended position at the neck of a user. The radiation-protective device (10) comprises: a head shield (12) and a support structure (80). The head shield (12) is a of sheet-like, pliable, and radiation attenuating first material. It has an outer edge (20) and an inner edge (22) and is arranged to position the inner edge (22) at the neck of the user in the intended position. The head shield (12) extends upwards from the inner edge (22) to the outer edge (20) in the intended position. The support structure (80) is attached to the head shield (12), deformable by hand, and arranged to bias the head shield (12) to have a desired shape in the intended position.

## Description

### Technical field

The technology proposed herein relates to wearable radiation-protective shields, and particularly to X-ray protective shields that have a support structure.

### Background

Ionizing radiation, such as X-ray, is commonly used in diagnostic and surgical procedures, such as in cardiology, or Interventional Radiology (IR). The clinical staff is exposed to the radiation during the procedures. Current standards and practices are based on the premise that any radiation dose may result in unfavourable health effects and can result in the development of radiation-induced diseases such as a specific type of cancer, glioblastoma. Reducing the radiation exposure of the clinical staff is therefore important.

The radiation can be direct or scattered. Radiation-protective shields, such as wearable radiation-protective garments including aprons, and thyroid collars, are used to reduce the exposure. There is a risk of radiation-induced damage or disease if the wearable radiation-protective garments are not worn, positioned, or oriented properly. This risk increases if the radiation-protective garments interfere with user's movements, or if the radiation-protective garments or shields does not properly fit the user.

### Object of the proposed technology

The proposed technology aims at increasing the comfort and freedom of motion for a user wearing a radiation-protective shield. It is a further object of the proposed technology to provide a radiation-protective device that protects the head and can be oriented and worn properly by users of different size and build. It is a further object of the proposed technology to provide a radiation-protective device with no, or reduced, impairment of the effective field of vision of a user.

### Summary

In a first aspect of the proposed technology, a wearable radiation-protective device, or shield, that has an intended position, or intended configuration, on, or at the neck or chest, of, a user is proposed. The radiation-protective device comprises: a head shield, upper shield part, and a support structure, or shaping element. The head shield is a of sheet-like, pliable, and radiation attenuating first material. The head shield has an outer edge, or first edge, and an inner edge, or second edge. The radiation-protective device positions, or is arranged to position, the inner edge, or the complete inner edge, at the neck or chest of the user in the intended position. The head shield extends upwards from the inner edge to the outer edge in the intended position. The support structure is attached to, or fixed to, the head shield. The support structure is deformable by hand.

It is specified that the radiation-protective device positions, or is arranged to position, the inner edge, or the complete inner edge, at the neck or chest of the user in the intended position. Worded differently, the inner edge, or the complete inner edge, is positioned at the neck or chest of the user in the intended position. It is further specified that the head shield extends upwards from the inner edge to the outer edge in the intended position. This means that the head shield protects at least parts of the neck and at lest parts of the head, or at least parts of the chest, the neck, and at least parts of the head, particularly if the radiation from a source in front of and below the head of the user.

The support structure may be plastically deformable. It is understood that this is under intended use of the radiation-protective device. It is further understood that the support structure is arranged to bias, or be able to bias, the head shield to have a desired shape, or a modified shape, in the intended position. Worded differently, the support structure is arranged to define the shape of the head shield in the intended position, or the head shield as such has an unmodified shape in the intended position, and the support structure is arranged to bias the head shield to have a modified shape, or a desired shape, in the intended position. Worded differently, the support structure is arranged to change the shape of the head shield at a deformation of the support structure, for example in the intended position.

It is understood that the head shield and the support structure, may be arranged to conform to the head of the user in the intended position.

It is specified that the support structure is deformable by hand. Worded differently, the support structure may be configured to deform plastically at a load that can be applied by hand. It is understood that this is without any tools. It is specified below that the support structure may be elongated. The load may be transverse to, or perpendicular to, to the elongated support structure. It is understood that the load may be localized, or more specifically at a load point. It is further understood that the load can be applied to the support structure as such, and/or via the head shield. It is further understood that the load can be applied to the support structure via the support cover and/or the tape further described below.

It is understood that the support structure may be configured to elastically deform at a first load on the support structure and plastically deform at a second load on the support structure. It is further understood that the second load is greater than the first load, or more specifically that the second load is caused by forces in the same direction but greater than the forces that causes the first load. With an elongated support structure, the first load and the second load may be transverse to, or perpendicular to, to the support structure. It is further understood that the support structure has a yield point between the first load and the second load.

In a second aspect of the proposed technology, a use of the wearable radiation-protective device according to the first aspect of the proposed technology is proposed for protecting a person, such as a clinical staff member, from radiation during a medical intervention comprising the use of radiation. Worded differently, a method of protecting a person, such as a clinical staff member, during a medical intervention comprising the use of radiation is proposed, wherein the method comprises: providing the wearable radiation-protective device according to the first aspect of the proposed technology; and placing, or positioning, the wearable radiation-protective device in the intended position on, or at the neck or chest, of, the clinical staff member.

The support structure may be of metal. It is understood that the metal may be an alloy, such as steel or nitinol. Alternatively, the support structure may be of plastic, such as high-density polyethylene. It is understood that the plastic may a reinforced plastic, such as glass-fibre reinforced polyethylene.

The support structure may be elongated. It may have a first end and an opposite second end. It is understood that the support structure extends between the first end and the second end. It is further understood that the support structure may be curved or bent in the intended position of the head shield. It is further understood that the elongated support structure may be arranged to deform at a load transverse, or perpendicular to the elongated support structure.

The support structure may comprise, or be, a wire, band, or strip. The support structure may be solid. For example, it may be a solid-core wire. Alternatively, the support structure may be hollow. For example, it may be a tube. The support structure may have a circular or square cross section. For example, it may be a wire with a circular or square cross section. Alternatively, the support structure may have a flattened, or rectangular, cross section, such as a band and a strip.

The radiation-protective device may comprise a cover that is deformable. It is understood that the cover may be elastically deformable at a deformation of the support structure. The cover may be arranged to space the support structure apart from adjacent parts of the radiation-protective device. The cover may be elongated. The cover may enclose at least parts of, or the complete, support structure. The cover may be sleeve and the support structure may be detached from, or loosely fitted within, the sleeve. The sleeve may be a tube. Alternatively, the cover may be a sheath that is attached to the support structure.

It is understood that the cover may be arranged to elastically deform at a deformation of the support structure. Worded differently, the cover may be elastically deformable. It is understood that this is under intended use of the radiation-protective device. The cover may contribute to reduce wear on parts of the radiation protective that are adjacent to the support structure, for example at a deformation of the support structure. This is particularly advantageous if the support structure is elongated and of metal, and if the adjacent parts are of fabric, or a fibrous material. The cover may be of an elastomer, for example rubber, such as silicone rubber. Alternatively, the cover may be of plastic, for example polyolefin, such as polyethylene.

It is understood that the head shield may have a forward end in the intended position relative to the user. The head shield may have a curved shape, or curved configuration, in the intended position and a planar shape, or planar configuration, if flattened on a planar surface, or at a flattening on a planar surface.

The head shield may have a first head-part section and a second head-part section that are divided by, or along, a first central line that extends normal to the inner edge and through the corresponding forward end in the planar shape of the head shield.

The outer edge may have a first end an opposite second end. The first end may be located on the first head-part section and the second end may be located on the second head-part section. Similarly, the inner edge may have a first end and an opposite second end. It is understood that the first end may be located on the first head-part section and the second end may be located on the second head-part section. It is understood that the head shield has a height h in the planar shape of the head shield between the inner edge and the outer edge and normal to inner edge.

It is understood that the outer edge may be located above, or at a level above, the inner edge in the intended position. For example, first head-part section may be on the right side of the first central line and the second head-part section may be on the left side of the first central line relative to the user, or vice versa. Worded differently, the first ends of the outer edge and the inner edge may be on the right side of the first central line, and the second ends of the outer edge and the inner edge may be on the left side of the first central line relative to the user, or vice versa.

It is specified that the device is radiation-protective. Expressed differently, the device protects against, or attenuates, ionizing radiation. The radiation-protective device may be an X-ray protective device, or shield. The radiation attenuating first material may be an X-ray attenuating first material. The X-ray attenuating first material may be arranged to attenuate X-ray radiation from a radiation source having a tube voltage of at least 40 kV, preferably at least 60 kV, and at the most 150 kV, preferably at the most 110 kV. For example, the X-ray attenuating first material may be arranged to attenuate X-ray radiation from a radiation source having a tube voltage in the range 40-150 kV, such as 60-110 kV. The X-ray attenuating first material may have at least 0.05 mm, preferably at least 0.10 mm, more preferably at least 0.25 mm, most preferably at least 0.50 mm, and at the most 1.00 mm, preferably at the most 0.75 mm, more preferably at the most 0.50 mm at any tube voltage in the range 60-110 kV. For example, the X-ray attenuating first material may have 0.05 to 1.00 mm Pb equivalence, preferably a 0.10 to 1.00 mm Pb equivalence, a 0.25 to 1.00 mm Pb equivalence, such as 0.25 mm to 0.75 mm Pb equivalence or 0.30 to 0.60 mm Pb equivalence, in particular at least 0.50 mm Pb equivalence, at any tube voltage in the range 60-110 kV. Here, Pb equivalence is to be measured according to the Inverse Broad Beam Geometry as described in IEC 61331-1:2014. The first material may be opaque, non-transparent or non-translucent to optical, or visible, light. This means that there essentially is no straight and unhindered path through the first material.

It is understood that in the intended position, the user is standing upright with the face facing straight forward.

It is understood that the forward end of the head shield may be defined by, or located at, the outer edge of the head shield. The radiation-protective device may be arranged to position the outer edge at the forward end at the chin of the user in the intended position. The radiation-protective device may be arranged to position the outer edge at the forward end at the level of the chin of the user in the intended position. The radiation-protective device may be arranged to position the outer edge at the forward end in front of the chin of the user in the intended position. It is understood that the head shield may be arranged to conform to the chin of the user in the intended position. Worded differently, the head shield may have a height at the forward end, or along the first central line, adapted to position the outer edge at the forward end at the chin, at the level of the chin, and/or in front of the chin of the user, in the intended position.

The outer edge may be located forward relative to the inner edge at the first central line in the intended position. Worded differently, the head shield may, or may be adapted to, extend upwards and outwards relative inner edge, or relative to the neck or chest of the user, at the forward end and in the intended position.

The head shield may have a first external angle (α) to a horizontal plane in the range 15° to 45°, 20° to 40°, or 25° to 35° at the forward end or the first central line. It is understood that this is in the intended position, for example with the user standing on the horizontal plane. It is further understood that the radiation-protective device is between the user and a point within the first external angle. It is further understood that the first external angle may be determined in a plane normal to the head shield at the forward end.

The radiation-protective device may be arranged to position the outer edge on the first head-part section on a first side of the neck or head of the user in the intended position. Similarly, the radiation-protective device may be arranged to position the outer edge on the second head-part section on a second side of the neck or head of the user in the intended position. It is understood that the first side and the second side are opposite relative to the neck or head of the user. The outer edge may be located above, or directly above, the inner edge at the first side of the neck or head of the user. The outer edge may be located at neck or head of the user at the first side of the neck or head of the user in the intended position. Similarly, the outer edge may be located above, or directly above, the inner edge at the second side of the neck or head of the user. The outer edge may be located at neck or head of the user at the second side of the neck or head of the user in the intended position.

The head shield may have a second external angle (β) to a horizontal plane in the range 70° to 100°, or 75 to 95 °, or 80° to 90, at the first side of the neck or head. Similarly, the head shield may have a second external angle (β) to a horizontal plane in the range 70° to 100°, or 75 to 95 °, or 80° to 90, at the second side of the neck or head. It is understood that this is in the intended position and that the user is standing on the horizontal plane. It is further understood that the radiation-protective device is between the user and a point within the second external angle. It is further understood that each second external angle may be determined in a plane normal to the head shield.

It is understood that the outer edge may outline an outer smooth curve in the planar shape of the head shield. It is further understood that the inner edge may outline an inner smooth curve in the planar shape of the head shield.

The features described here contributes to the head shield extending more outwards at the throat than at the sides of the neck or head. This contributes to reduce radiation exposure to the eyes and the brain, for example in IR applications, with little impairment of the effective field of vision of the user.

The outer edge may have a first outer-edge section that extends from the first central line. It is understood that it extends to a point between the first central line and the first end of the outer edge. The first outer-edge section may be straight or convex in the planar shape of the head shield. The outer edge may have a second outer-edge section that extends from the first outer-edge section. It is understood that it extends to a point between the first outer-edge section and the first end of the outer edge. The second outer-edge section may be concave in the planar shape of the head shield. The outer edge may have a third outer-edge section that extends from the second outer-edge section. It is understood that it may extend to the first end of the outer edge, or to a point between the second outer-edge section and the first end of the outer edge. The third outer-edge section may be convex in the planar shape of the head shield. This contributes to position the outer edge forward below and at the side of the eyes, which improves the radiation protection of the eyes, for example in IR applications, with little impairment of the forward field of vision of the user.

The head shield may have a first side edge, or third edge, that interconnects the outer edge and the inner edge, or more specifically the first end of the outer edge and the first end of the inner edge. Similarly, the head shield may have a second side edge, or fourth edge, that interconnects the outer edge and the inner edge, or more specifically the second end of the outer edge and the second end of the inner edge. The first side edge and/or the second side edge may be straight. The first side edge and/or the second side edge head shield may have a third external angle (γ) to a horizontal plane in the range 60° to 100°, or 70 to 95°, or 80° to 90°. It is understood that this is in the intended position, for example with the user standing on the horizontal plane. It is further understood that a point within each third external angle is located outside the head shield. The first side edge and/or the second side edge may be located at the neck or head of the user in the intended position. Worded differently, the radiation-protective device may be arranged to position the first side edge and/or the second side edge at the neck or head of the user in the intended position. The features described here contributes to improve the radiation protection of the eyes, for example in IR applications, with no impairment of the field of vision of the user.

Each of the first side edge and the second side edge may have a curved section at the outer edge, or at the first end respectively the second end of the outer edge. For example, the curved section may outline an arc of a circle with a radius that is greater than 5 mm, 10 mm, or 15 mm. The radius may be smaller than 20 mm, 25 mm, or 30 mm.

The inner edge may have a first central-edge section that extends from the first central line to a point between the first central line and the first end of the inner edge. The inner edge may have a first peripheral-edge section that is located between the first central-edge section and the first end of the inner edge. It is understood that the first central-edge section and the first peripheral-edge section may be disjoint.

The inner edge may have a second central-edge section that extends from the first central line to a point between the first central line and the second end of the inner edge. The inner edge may have a second peripheral-edge section that is located between the second central-edge section and the second end of the inner edge. It is understood that the second central-edge section and the second peripheral-edge section may be disjoint.

The outer edge on the first head-part section may be between 1.8 and 1.2, 1.7 and 1.3, or 1.6 and 1.4, times longer than the inner edge on the first head-part section.

The first side edge may have a length that is longer than 0.7, 0.8, or 0.9 times the height at the first central line. The first side edge may have a length that is shorter than 1.2, 1.1, or 1.0, times the height at the first central line. Similarly, the second side edge may have a length that is longer than 0.7, 0.8, or 0.9, times the height at the first central line. The second side edge may have a length that is shorter than 1.2, 1.1, or 1.0, times the height at the first central line.

It has been found that the features described here significantly improves the radiation protection of the eyes and the brain, for example in IR applications, with little additional impairment of the field of vision of the user.

The first central-edge section may extend to, or at least to, a point having an arc-length separation from the first central line of 3cm, 4 cm, or 5 cm. Similarly, the second central-edge section may extend to, or at least to, a point having an arc-length separation from the first central line of 3cm, 4 cm, or 5 cm.

The first peripheral-edge section may extend from, or at least from, a point having an arc-length separation from the first central line of 4 cm, 5 cm, or 6 cm. Similarly, the second peripheral-edge section may extend from, or at least from, a point having an arc-length separation from the first central line of 4 cm, 5 cm, or 6 cm.

The first peripheral-edge section may extend to, or at least to, a point having an arc-length separation from the first central line of 13 cm, or 15 cm. Similarly, the second peripheral-edge section may extend to, or at least to, a point having an arc-length separation from the first central line of 13 cm, or 15 cm. The first peripheral-edge section may extend to the first end of the inner edge. Similarly, the second peripheral-edge section may extend to the second end of the inner edge.

The inner edge on the first head-part section may be at least 13 cm, or 15 cm, in length. Worded differently, the inner edge on the first head-part section may have an arc length of at least 13 cm, or 15 cm. Similarly, the inner edge on the second head-part section may be at least 13 cm, or 15 cm, in length. Worded differently, the inner edge on the second head-part section may have an arc length of at least 13 cm, or 15 cm.

The height at the forward end, or along the first central line, may be greater than 3 cm, greater than 4 cm, or greater than 5 cm. The height at the forward end, or along the first central line, may be smaller than 8 cm, smaller than 9 cm, or smaller than 10 cm.

It has been found that the features described here significantly improves the radiation protection of the eyes, for example in IR applications, with little additional impairment of the field of vision of the user.

The head shield may be symmetric relative to the first central line in the planar shape of the head shield. More specifically, the first head-part section and the second head-part section may have a reflection symmetry relative to the first central line in the planar shape of the head shield. It is specified that the head shield has a first head-part section and a second head-part section that are divided by a first central line. Worded differently, the head shield may have a bisecting first central line that extends normal to the inner edge and through the corresponding forward end in the planar shape of the head shield, wherein the first central line divides the head shield in a first head-part section and a second head-part section that are congruent. It is understood that the congruent sections have shapes that are transformable to one another by reflection and/or rotation. The symmetry contributes to an even load on the user, which in extension contributes to a more comfortable radiation-protective device.

The first material may be, or comprise, a permeable fabric, or fibrous material. The permeable fabric may comprise filaments. The filaments may be arranged in a regular pattern. The filaments may extend in a uniform direction. The uniform direction may be aligned with, or parallel with, the first central line. Worded differently, the uniform direction may be transverse to, or perpendicular to, the outer edge at the forward end. This contributes to a wearable radiation-protective shield that is comfortable and form stable. It is understood that the first material may be permeable to gas, for example air and water vapour. Worded differently, the first material may be gas-permeable .

Each filament may be of a solid composition that comprises a carrier substance and a radiopaque substance. For example, the solid composition may be a polymeric composite material. The filaments may be arranged in a regular pattern. It is understood that each filament may be a monofilament. It is further understood that the carrier substance and the radiopaque substance may be mixed, and that the radiopaque substance may be evenly distributed within the carrier substance. The carrier substance may comprise at least one polymer and the radiopaque substance may comprise at least one metal. For example, the polymer may be a thermoplastic polymer such as polyvinyl chloride or a polyolefin, and the metals may, as a powder, be as an element, oxide or alloy of for example Lead, Barium, Antimony, Bismuth, or Tungsten(Wolfram), and any combination thereof..

The fabric may be a woven fabric with the filaments forming the weft of the fabric. It is understood that the fabric has a warp, for example of polyester. More specifically, the first material may be any of the radiation-protective material described in WO2014163574A1. The fabric contributes to a more comfortable radiation-protective device.

Alternatively, the first material may be, or comprise, an impermeable sheet. The impermeable sheet may be of a solid composition that comprises a carrier substance and a radiopaque substance. For example, the solid composition may be a polymeric composite material. It is further understood that the carrier substance and the radiopaque substance may be mixed, and that the radiopaque substance may be evenly distributed within the carrier substance. The carrier substance may comprise at least one polymer and the radiopaque substance may comprise at least one metal.

It is understood that the first material may be composed of several layers, for example two layers of fabric or two impermeable sheets.

The radiation-protective device may comprise: a head-part lining, or upper part-lining, that covers the head shield, or prevent direct access to the head shield, wherein the head-part lining is of sheet-like and pliable first lining material. It is understood that the first lining material may be non-attenuating for radiation. For example, the first lining material may be of non-breathable polyurethane or of a fabric of polyester with water-proof breathable membrane.

The radiation-protective device, or more precisely the head shield, may have a first attachment part, or upper attachment part, at the inner edge arranged to cooperate with a second attachment part to releasably attach the head shield to the second attachment part. For example, the first attachment part and the second attachment part may be a hook-and-loop fastener, or the second attachment part may form a slot into which the first attachment part is inserted. For example, the second attachment part may form part of a radiation-protective neck shield or garment.

The head shield may be composed of a single piece of the first material. Alternatively, the head shield may be composed of a plurality of pieces of the first material. The pieces may be overlapping. It is understood that the pieces may be joined together. For example, they may be joined together by sewn seams, or construction stiches. Alternatively, the overlapping pieces may be welded or glued together.

It is understood that the head shield may have an outer side surface and an inner side surface, wherein the outer side surface faces away from the user and the inner side surface faces towards the user in the intended position of the radiation-protective device. The head-part lining may be located at the inner side surface and/or at the outer side surface of the head shield. The head-part lining may cover, or fold over, the outer edge, first side edge, and/or second side edge. More specifically, the head-part lining may have, or form, a fold that covers, or fold overs, the outer edge, first side edge, and/or second side edge. It is understood that this may be over the complete edges, or over parts of the edges. The the head-part lining may overlap itself, for example at the outer edge of the head shield.

The support structure may be centred between the outer side surface and the inner side surface. Alternatively, the support structure may be located on the outer side surface or the inner side surface of the head shield, preferably the former.

The support structure may be attached to, or fixed to, the head shield at the outer edge, first side edge, and/or second side edge of the head shield. It may further be attached to, or fixed to, the neck shield further specified below, for example at the first upper edge of the neck shield. The support structure may be located at and/or extend along the outer edge, first side edge, and/or second side edge of the head shield. It may further be located at and/or extend along the first upper edge of the neck shield. The support structure may be located at and/or extend along the complete edges, or over parts of the edges.

It is understood that there may be a connection between the outer edge and the first side edge, and the support structure may extend across the connection, there may be a connection between the outer edge and the second side edge, and the support structure may extend across the connection, there may be a connection between the first side edge and the first upper edge, and the support structure may extend across the connection, and there may be a connection between the second side edge and the first upper edge, and the support structure may extend across the connection. It is further understood that the support structure may be bent at any of the connections.

It is understood that the head shield may have a first side and an opposite second side relative to the forward end in the intended position of the radiation-protective device. For example, the first side may be the right side, and the second side may be the left side in the intended position. It is specified that the head shield may have has a first head-part section and a second head-part section, and it is understood that the first head-part section may be on the first side and the second head-part section may be on the second side.

The support structure may be located on the first side and the second side of the head shield. It is understood that the support structure may extend from the first side to the second side. It is specified above that the support structure may be elongated, and that it may have a first end and an opposite second end. The first end and the second end of the support structure may be located on opposite sides of the head shield relative to the forward end in the intended position of the radiation-protective device. More specifically, the first end of the support structure may be located on the first side and the second end of the support structure may be located on the second side. It is understood that the support structure may be located at, or extend across, the forward end of the head shield.

The first end of the support structure may be located at the outer edge of the head shield. It may be located between the first end of the outer edge and the forward end. Preferably, it may be located closer to the first end of the outer edge than to the forward end. It is understood that this may be when the support structure extends along a part of the outer edge. The first end of the support structure may be located at the first end of the outer edge of the head shield, or at a connection between the first side edge and the outer edge. It is understood that this may be when the support structure extends along the complete outer edge.

Alternatively, the first end of the support structure may be located at the first side edge of the head shield. It may be located between the first end of the outer edge and the first end of the inner edge. For example, it may be located closer to the first end of the inner edge than to the first end of the outer edge. It is understood that this may be when the support structure extends along the complete outer edge and a part of the first side edge. The first end of the support structure may be located at the first end of the inner edge of the head shield, or at a connection between the first side edge and the inner edge. It is understood that this may be when the support structure extends along the complete outer edge and the complete first side edge. It is further understood that the support structure may extend across a connection between the first upper edge and the first side edge, and that it may be bent at the connection.

Alternatively, the first end of the support structure may be located at the first upper edge of the neck shield further specified below. The first end of the support structure may be spaced apart from, or distant from, the first end of the inner edge, the first side edge, or a connection between the first side edge and the first upper edge. The support structure may extend away from the first end of the inner edge. For example, it may extend forwards, or towards the front end of the radiation-protective device, or it may extend rearwards, or away from the front end of the radiation-protective device. It is understood that this may be when the support structure extends along the complete outer edge, the complete first side edge, and a part of the first upper edge. It is further understood that the support structure may extend across a connection between the first side edge and the first upper edge, and that it may be bent at the connection.

The second end of the support structure may be arranged relative to the forward end, the outer edge, the second side edge, respective the first upper edge as described above for the first end of the support structure relative to the forward end, the outer edge, the first side edge, respective the first upper edge.

Alternatively to the support structure being located on the first side and the second side of the head shield, the support structure may be located on the first side of the head shield and spaced apart from, or distant from, the second side of the head shield, or vice versa. More specifically, the first end and the second end of the support structure may be located on the first side of the head shield and spaced apart from, or distant from, the second side of the head shield, or vice versa. Worded differently, the first end and the second end of the support structure may both be located on the first side, or they may both be located on the second side.

The second end of the support structure may be located at the outer edge of the head shield. It may be located between the first end of the outer edge and the forward end. It may be located closer to the first end of the outer edge than to the forward end, or vice versa. It is understood that this may be when the support structure extends along a part of the outer edge.

The first end of the support structure may then be located at the outer edge of the head shield. It may be located between the second end of the support structure and the first end of the outer edge. For example, it may be located closer to the first end of the outer edge than to the forward end. It is understood that this may be when the support structure extends along a part of the outer edge. The first end of the support structure may be located at the first end of the outer edge of the head shield, or at a connection between the first side edge and the outer edge. It is understood that this may be when the support structure extends along a part of the outer edge.

Alternatively, the first end of the support structure may be located at the first side edge of the head shield. It may be located between the first end of the outer edge and the first end of the inner edge. For example, it may be located closer to the first end of the inner edge than to the first end of the outer edge. It is understood that this may be when the support structure extends along a part of the outer edge and a part of the first side edge. The first end of the support structure may be located at the first end of the inner edge of the head shield, or at a connection between the first side edge and the inner edge. It is understood that this may be when the support structure extends along a part of the outer edge and the complete first side edge. It is further understood that the support structure may extend across a connection between the first upper edge and the first side edge, and that it may be bent at the connection.

Alternatively, the first end of the support structure may be located at the first upper edge of the neck shield further specified below. It may be spaced apart from, or distant from, the first end of the inner edge, the first side edge, or a connection between the first side edge and the first upper edge. At the first upper edge, the support structure may extend away from the first end of the inner edge of the head shield. For example, it may extend forwards, or towards the front end of the radiation-protective device, or it may extend rearwards, or away from the front end of the radiation-protective device. It is understood that this may be when the support structure extends along a part of the outer edge, the complete first side edge, and a part of the first upper edge. It is further understood that the support structure may extend across a connection between the first side edge and the first upper edge, and that it may be bent at the connection.

The second end of the support structure may be located at the first end of the outer edge of the head shield, or at a connection between the first side edge and the outer edge. It is understood that this may be when the support structure extends along a part of the outer edge. Alternatively, the second end of the support structure may be located at the first side edge of the head shield. It may be located between the first end of the outer edge and the first end of the inner edge. Preferably, it may be located closer to the first end of the outer edge than to the first end of the inner edge. It is understood that this may be when the support structure extends or a part of the first side edge. The second end of the support structure may be located at the first end of the outer edge of the head shield, or at a connection between the first side edge and the outer edge. It is understood that this may be when the support structure extends along the complete first side edge.

The first end of the support structure may then be located at the first side edge of the head shield. It may be located between the first end of the outer edge and the first end of the inner edge. Preferably, it may be located closer to the first end of the inner edge than to the first end of the outer edge. It is understood that this may be when the support structure extends along a part of the first side edge. The first end of the support structure may be located at the first end of the inner edge of the head shield, or at a connection between the first side edge and the inner edge. It is understood that this may be when the support structure extends along the complete first side edge.

Alternatively, the first end of the support structure may be located at the first upper edge of the neck shield further specified below. It may be spaced apart from, or distant from, the first end of the inner edge, the first side edge, or a connection between the first side edge and the first upper edge. The support structure may extend away from the first side edge, away from the first end of the inner edge, or away from a connection between the first side edge and the first upper edge. For example, it may extend forwards, or towards the front end of the radiation-protective device, or it may extend rearwards, or away from the front end of the radiation-protective device. It is understood that this may be when the support structure extends along a part of or the complete first side edge, and a part of the first upper edge. It is further understood that the support structure may extend across a connection between the first side edge and the first upper edge, and that it may be bent at the connection.

It is specified above that the support structure may be elongated, and that it may have a first end and an opposite second end. It is further specified above that the support structure may be attached to, or fixed to, the head shield at the outer edge, first side edge, and/or second side edge of the head shield. Alternatively, the support structure may extend between the inner edge and the outer edge of the head shield. More specifically, the support structure may extend from a point at the inner edge to a point at outer edge. The support structure may extend transverse to the inner edge and/or the outer edge. The support structure may be attached to, or fixed to, the head shield at the inner edge and the outer edge of the head shield. Additionally or alternatively, the support structure may be attached to, or fixed to, the head shield between the inner edge and the outer edge, or along the support structure.

It is understood that the support structure may be located on the first side or the second side of the head shield, or at the first head-part section or the second head-part section of the head shield. Preferably, the support structure may be located at the outer side surface of the head shield. This way, the head shield is located between the support structure and the user, which means that the support structure as such has less effect on comfort. Alternatively, it may be located at the inner side surface of the head shield. This allows the support structure to push the head shield away from the user, which means that less arrangements for attaching, or fixing, the support structure to the head shield are required.

The support structure may be spaced apart from, or distant from, the first side edge and/or the second side edge of the head shield. More specifically, the support structure may be spaced apart from, or distant from, the first end and the second end of the outer edge, and/or it may be spaced apart from, or distant from, the first end and the second end of the inner edge. The support structure may be spaced apart from, or distant from, the forward end of the radiation-protective device. The support structure may be located closer to the first side edge or the second side edge of the head shield than to the forward end of the radiation-protective device. Worded differently, the support structure may be located closer to the first end of the outer edge and/or the first end of the inner edge than to the forward end, or closer to the second end of the outer edge and/or the second end of the inner edge than to the forward end.

It is specified above that the inner edge may have a first central-edge section, a first peripheral-edge section, a second central-edge section, and a second peripheral-edge section. The support structure may be located at the first peripheral-edge section or the second peripheral-edge section.

It is specified that the support structure is attached to, or fixed to, the head shield. More specifically, the head-part lining may attach, or fix, the support structure to the head shield. Worded differently, the head-part lining may be arranged to bias the head shield relative to, or towards, the support structure, or vice versa. It is understood that this may be at a deformation of the support structure. The head-part lining may be attached to, or fixed to, the head shield at the support structure.

At least a part of, or the complete, support structure may be located between the head-part lining and the head shield. The head-part lining may be attached to, or fixed to, the head shield on both sides of the support structure. For example, the head-part lining may be attached to, or fixed to, the head shield by one or more seams on both sides of the support structure, and the head-part lining and the one or more seams may jointly attach, or fix, the support structure to the head shield. It is understood that the one or more seams may be disjoint from the support structure. This means that the seams do not cross the support structure. The one or more seams may extend along, or parallel with, the support structure.

The one or more seams may be sewn seams, or construction stiches. Alternatively, the one or more seams may be weld seams or adhesive seams. If the first material comprises filaments, this may have less effect on the relative positions of the filaments than the sewn seams.

It is understood that the attachment involving the head-part lining may be with the support structure extending between the inner edge and the outer edge of the head shield, but also with the support structure being attached to the head shield at the outer edge, first side edge, and/or second side edge of the head shield.

It is specified above that the head-part lining may have a fold that covers the outer edge, first side edge, and/or second side edge. The fold may cover the support structure at respective edge. The fold may may attach, or fix, the support structure to the head shield. It is understood that this may be for at least a part of, or the complete support structure. Worded differently, the fold may be arranged to bias the head shield relative to, or towards, the support structure, or vice versa. It is understood that this may be at a deformation of the support structure. For example, the head-part lining may be attached to, or fixed to, the head shield by one or more seams on both sides of the support structure, and the head-part lining and the one or more seams may jointly attach, or fix, the support structure to the head shield.

It is understood that the support structure may be located at the fold. It is specified above that the support structure may be elongated, and it is understood that the fold may be elongated and aligned with, or parallel with, the support structure. The fold may cover, or fold over, at least parts of, or the complete, support structure.

The radiation-protective device may further comprise: a tape that is attached to, or fixed to, the head shield, for example at the outer edge, first side edge, and/or second side edge. It is understood that the tape is pliable. The tape may cover, or fold over, the outer edge, first side edge, and/or second side edge. It is understood that this may be over the complete edges, or over parts of the edges. It is further understood that the tape may overlap the head shield, and that it may overlap the head-part lining if present. In the latter case, it is understood that the head-part lining may be located between the tape and the head shield. The tape may cover, or fold over, the first upper edge of the neck shield further specified below. It is understood that this may be over parts of the first upper edge. It is understood that the tape may overlap the neck shield, and that it may overlap the neck-part lining if present. In the latter case, it is understood that the neck-part lining may be located between the tape and the neck shield. It is understood that the tape may be composed of a single piece of the tape, or that it may be composed of a plurality of tape sections. It is further understood that the tape may be aligned with any of the abovementioned edges.

It is specified that the support structure is attached to, or fixed to, the head shield. More specifically, the tape may attach, or fix, the support structure to the head shield. It is understood that this may be for at least a part of, or the complete support structure. Worded differently, the tape may be arranged to bias the head shield relative to, or towards, the support structure, or vice versa. It is understood that this may be at a deformation of the support structure.

It is understood that the tape may be located at the support structure, or vice versa. It is specified above that the support structure may be elongated, and it is understood that the tape may be elongated and aligned with, or parallel with, the support structure. The tape may cover, or fold over, the support structure. It is understood that this may for at least parts of, or the complete, support structure.

The tape may be attached to, or fixed to, the head shield on both sides of the support structure, for example if the support structure extends between the inner edge and the outer edge of the head shield. It is understood that the tape has opposite longitudinal sides. The longitudinal sides may be located on opposite sides of the support structure, for example if it covers the support structure and the support structure extend between the inner edge and the outer edge of the head shield. Alternatively, the longitudinal sides may be located on the same side of the support structure, for example if it folds over the support structure and the support structure is located at and extend along the outer edge, first side edge, and/or second side edge of the head shield.

The tape may be attached to, or fixed to, the head shield and/or the neck shield specified below by one or more seams. For example, the tape may be a bias tape, or bias binding, such as a fabric tape. The tape and the one or more seams may jointly attach, or fix, the support structure to the head shield and/or the neck shield. It is understood that the one or more seams may be disjoint from the support structure. The one or more seams may extend along, or parallel with, the support structure. Additionally or alternatively, the tape may be attached to, or fixed to, the head shield and/or the neck shield specified below by an adhesive. For example, the tape may be an adhesive tape.

The radiation-protective device may further comprise: a neck shield, middle shield part, wherein the neck shield is a of sheet-like, pliable, and radiation attenuating second material. The neck shield is adapted to extend partly, at least partly, or completely around the neck of the user in the intended position. The head shield is connected to, or attached to, the neck shield, for example at the inner edge of the head shield. It is understood that the neck shield as such may have a curved shape, or curved configuration, in the intended position and a planar shape, or planar configuration, if flattened on a planar surface, or at a flattening on a planar surface. It is specified that the neck shield is adapted to extend at least partly around the neck of the user in the intended position. Worded differently, the neck shield may be adapted to extend from, or at least from, a first side of the neck of the user, across the front of the neck of the user, and to an opposite second side of the neck of the user.

It is understood that it may not be possible to simultaneously flatten the head shield and the neck shield on a planar surface with the head shield connected to the neck shield. It is further understood that the head shield may extend upwards relative to the neck shield, in the intended position. It is further understood that the neck shield, may be arranged to conform to the neck of the user in the intended position. It is further understood that the head shield and the support structure may be fully supported by the neck shield in the intended position.

The radiation-protective device may have a neck-part extension that is connected, or attached, to and extends from the neck shield. The neck shield and the neck-part extension may be arranged to jointly encircle, or extend completely around, the neck of the user in the intended position. The neck-part extension may be an extension of the neck-part lining described below.

The neck-part extension may have an overlap in the intended position, for example at the back of the neck of the user. The radiation-protective device, or the neck-part extension, may have a lock at the overlap arranged to releasably lock, or fixate, the neck shield around the neck of the user in the intended position. For example, the lock may comprise a hook-and-loop fastener or cooperating magnets.

For example, the head shield, may be connected to the neck shield by a sewn seam, or a construction stich. Alternatively, the head shield may be welded or glued to the neck shield.

The neck shield may be elongated, or tape-like structure. The neck shield may have a first upper edge, or fifth edge. It may further have a first lower edge, or sixth edge. The first upper edge may outline a straight line in the planar shape of the neck shield. Similarly, the first lower edge may outline a straight line in the planar shape of the neck shield. The first upper edge and first lower edge may be aligned, or parallel.

The neck shield may be arranged to position the outer edge at the forward end of the head shield at the chin at the level of the chin, and/or in front of the chin in the intended position.

The neck shield may outline a cylindrical geometry in the intended position. The cylindrical geometry may have a cylinder axis that has an angle (δ) to a horizontal plane at 65°, or in the range 55° to 75°, or 60° to 70°. It is understood that this is in the intended position, for example with the user standing on the horizontal plane.

The inner edge, or the complete inner edge, of the head shield may be located at the first upper edge of the neck shield. The head shield and the neck shield may overlap at the inner edge, or along the complete inner edge. The head shield and the neck shield may overlap by less than 10 mm, or less than 5 mm.

It is specified that the radiation-protective device may be arranged to position the outer edge on the first head-part section on a first side of the neck or head of the user. More specifically, the neck shield may be arranged to position the outer edge on the first head-part section on a first side of the neck or head of the user. Similarly, it is specified that the radiation-protective device may be arranged to position the outer edge on the first head-part section on a second side of the neck or head of the user. More specifically, the neck shield may be arranged to position the outer edge on the second head-part section on a second side of the neck or head of the user.

The head shield may have a fourth external angle (ε) to neck shield at the forward end, or the first central line, that is in the range 130° to 160°, 135° to 155°, or 140° to 150°. It is understood that the fourth external angle may be in a plane normal to the head shield. It is further understood that the radiation-protective device is between the user and a point within the fourth external angle.

The shape of the head shield may conform to the shape of the neck shield on the first side of the neck or head of the user in the intended position. Similarly, the shape of the head shield may conform to the shape of the neck shield on the second side of the neck or head of the user in the intended position. The head shield may have a fifth external angle (ζ) to the neck shield in the range 160° to 190°, 165° to 185°, or 170° to 180°, at the first side of the neck or head. Similarly, the head shield may have a fifth external angle (ζ) to the neck shield in the range 160° to 190°, 165° to 185°, or 170° to 180°, at the second side of the neck or head. It is understood that each fifth external angle may be in a plane normal head shield. It is further understood that the radiation-protective device is between the user and a point within each fifth external angle.

The radiation-protective device, or the neck shield, may position the first side edge and/or the second side edge at the neck or head of the user in the intended position. The first side edge and/or the second side edge of the head shield may have a sixth external angle (η) to the first upper edge of the neck shield that is in the range 35° to 75°, 45° to 70°, or 55° to 65°. It is understood that a point within the sixth external angle is located outside the head shield and the neck shield. It is further understood that the sixth external angle is relative to the head shield at the respective side edge.

It is understood that the first upper edge of the neck shield may be longer than the inner edge of the head shield. It is specified that the neck shield may be elongated. The neck shield may have a length, or average length, in the planar shape of the neck shield in the range 35 cm to 50 cm, or 40 cm to 45 cm. It is understood that the length may be along, or parallel with, the first upper edge and/or the first lower edge. The neck shield may have a width, or an average width, in the planar shape of the neck shield in the range 3.0 cm to 5.0 cm, or 3.5 to 4.5 cm. It is understood that the width may be transverse to, or perpendicular to, the first upper edge and/or the first lower edge. The combined neck shield and neck-part extension may have a length in the range 55 cm to 70 cm, or 60 to 65 cm.

Details of the first material are described above. The second material may have any of the features of the first material described above. For example, the second material may be, or comprise, a permeable fabric, or fibrous material. The permeable fabric may comprise filaments. The filaments may be arranged in a regular pattern. The filaments may extend in a uniform direction. The uniform direction may be transverse to, or perpendicular to, the first upper edge and/or the first lower edge of the neck shield. This contributed to a stable yet comfortable wearable radiation-protective shield. The second material may be the same as the first material.

The radiation-protective shield may comprise: a neck-part lining, or middle part-lining, that covers the neck shield, or prevent direct access to the neck shield, wherein the neck-part lining is of sheet-like and pliable second lining material. It is understood that the second lining material may be non-attenuating to radiation. For example, the second lining material may be of non-breathable polyurethane or a of fabric of polyester with water-proof breathable membrane. The second lining material may be the same as the first lining material.

The neck shield may be composed of a single piece of the second material. Alternatively, the neck shield may be composed of a plurality of overlapping pieces of the second material. The overlapping pieces may be joined together. For example, they may be joined together by sewn seams, or construction stiches. Alternatively, the overlapping pieces may be welded or glued together.

It is specified that the head shield may be connected to, or attached to, the neck shield at the inner edge of the head shield. The radiation-protective device, or more precisely the neck shield, may have a first attachment part arranged to cooperate with a second attachment part to releasably attach the neck shield to the second attachment part. For example, the first attachment part and the second attachment part may form a hook-and-loop fastener, or the second attachment part may form a slot into which the first attachment part is inserted. For example, the second attachment part may form part of a radiation-protective chest shield or garment.

The radiation-protective device may further comprise: a chest shield, lower shield part, wherein the chest shield is a of sheet-like, pliable, and radiation attenuating third material. The chest shield is adapted to cover an upper part of the chest at the front of the user in the intended position. The neck shield is connected to, or attached to, the chest shield, for example at the first lower edge of the neck shield. It is understood that the chest shield as such may have a curved shape, or curved configuration, in the intended position and a planar shape, or planar configuration, if flattened on a planar surface, or at a flattening on a planar surface.

The neck shield and the chest shield may jointly form a thyroid shield, or thyroid collar. It is understood that it may not be possible to simultaneously flatten the head shield, the neck shield, and the chest shield on a planar surface with the parts connected. It is further understood that the chest shield may extend downwards relative to the neck shield in the intended position. It is further understood that the chest shield may be arranged to conform to the chest of the user in the intended position. It is further understood that the chest shield may be fully supported by the neck shield in the intended position.

The chest shield may have a second upper edge, or seventh edge, and a second lower edge, or eighth edge. The second upper edge of the chest shield may be concave in the planar shape of the chest shield. The second lower edge of the chest shield may be convex in the planar shape of the chest shield. It is understood that the second upper edge of the chest shield may outline an upper smooth curve in the planar shape of the chest shield. It is further understood that the second lower edge may outline a lower smooth curve in the planar shape of the chest shield.

The second upper edge, or the complete second upper edge, of the chest shield may be located at the first lower edge of the neck shield. The chest shield and the neck shield may overlap at the second upper edge, or along the complete second upper edge, of the chest shield. The chest shield and the neck shield may overlap by less than 10 mm, or less than 5 mm.

For example, the chest shield may be connected to the neck shield by a sewn seam, or a construction stich. Alternatively, the chest shield may be welded or glued to the neck shield.

The chest shield may have a first chest-part section and a second chest-part section that are divided by, or along, a second central line that extends normal to the second upper edge of the chest shield, and the chest shield is symmetric relative to the second central line in the planar shape of the chest shield. More specifically, the first chest-part section and a second chest-part section may have a reflection symmetry relative to the second central line in the planar shape of the chest shield. Worded differently, the chest shield may have a bisecting second central line that extends normal to the second upper edge of the chest shield in the planar shape of the chest shield, wherein the second central line divides the chest shield in a first chest-part section and a second chest-part section that are congruent. It is understood that the congruent sections have shapes that are that are transformable to one another by reflection and/or rotation.

The chest shield has a height in the planar shape of the chest shield between the second upper edge and the second lower edge of the chest shield and normal to second upper edge of the chest shield. The height along the second central line, may be greater than 8 cm, greater than 9 cm, or greater than 10 cm. The height at the second central line, may be smaller than 12 cm, smaller than 14 cm, or smaller than 16 cm.

Details of the first material are described above. The third material may have any of the features of the first material described above. For example, the third material may be, or comprise, a permeable fabric, or fibrous material. The permeable fabric may comprise filaments. The filaments may be arranged in a regular pattern. The filaments may extend in a uniform direction. The uniform direction may be transverse to, or perpendicular to, the second central line. Worded differently, the uniform direction may be aligned with, or parallel with, the second lower edge of the chest shield at the centre of the second lower edge and in the planar shape of the chest shield.

The third material may be the same as the first material and/or the second material.

The radiation-protective device may comprise: a chest-part lining, or lower part-lining, that covers the chest shield, or prevent direct access to the chest shield, wherein the chest-part lining is of sheet-like and pliable third lining material. It is understood that the third lining material may be non-attenuating to radiation. For example, the third lining material may be of non-breathable polyurethane or a of fabric of polyester with water-proof breathable membrane. The third lining material may be the same as the first lining material and/or the second lining material.

The chest shield may be composed of a single piece of the third material. Alternatively, the chest shield may be composed of a plurality of overlapping pieces of the third material. The overlapping pieces may be joined together. For example, they may be joined together by sewn seams, or construction stiches. Alternatively, the overlapping pieces may be welded or glued together.

### Brief description of the drawings

A more complete understanding of the abovementioned and other features and advantages of the technology proposed herein will be apparent from the following detailed description of preferred embodiments in conjunction with the appended drawings, wherein:
**Figs. 1a and 1b** show an embodiment of a radiation-protective device in an intended position on a user.
**Figs. 2a to 2d** are different views of the radiation-protective device of Figs. 1a and 1b.
**Fig. 3** shows the head shield of the radiation-protective device of Figs. 1a and 1b.
**Fig. 4** shows the neck shield of the radiation-protective device of Figs. 1a and 1b in the same scale as in Fig. 3.
**Fig. 5** shows the chest shield of the radiation-protective device of Figs. 1a and 1b in the same scale as in Fig. 3.
**Figs. 6a to 6b** are different views of the radiation-protective device of Figs. 1a and 1b with the support structure revealed.
**Fig. 7** is a view of another embodiment of a radiation-protective device.
**Fig. 8** is a view of another embodiment of a radiation-protective device.
**Fig. 9** is a view of another embodiment of a radiation-protective device.
**Fig. 10** is a view of another embodiment of a radiation-protective device.
**Figs. 11a to 11e** show are cross sections at the support structure of different embodiments of the radiation-protective device.

### Detailed description

In the figures and the below description, the same reference numerals are used for the same or related features.

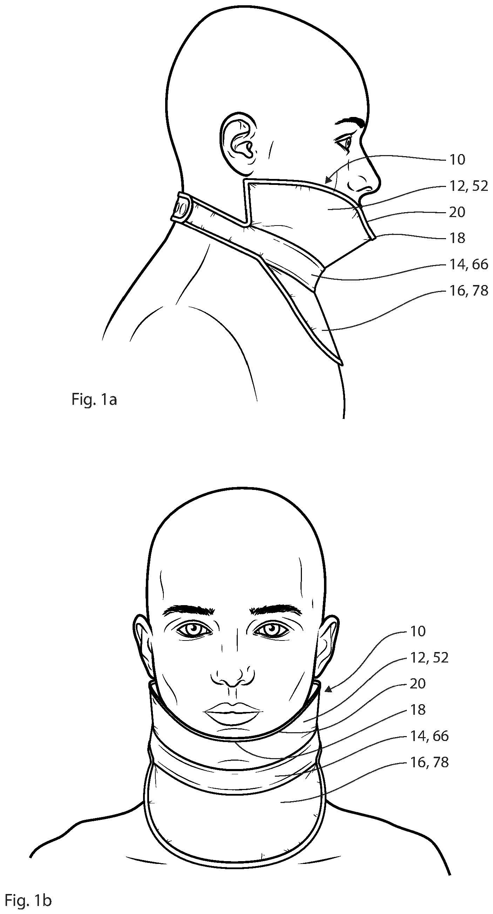

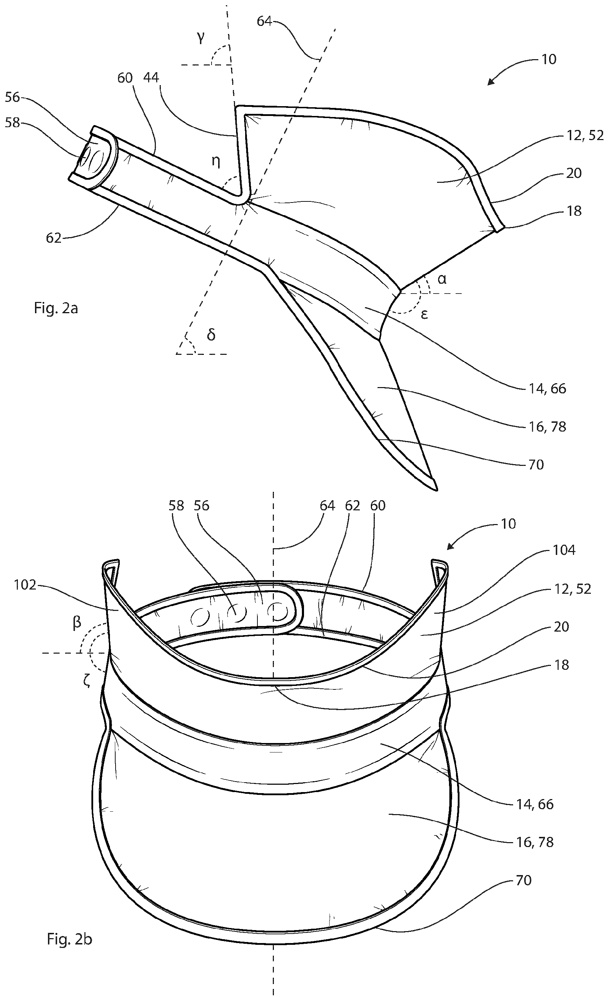

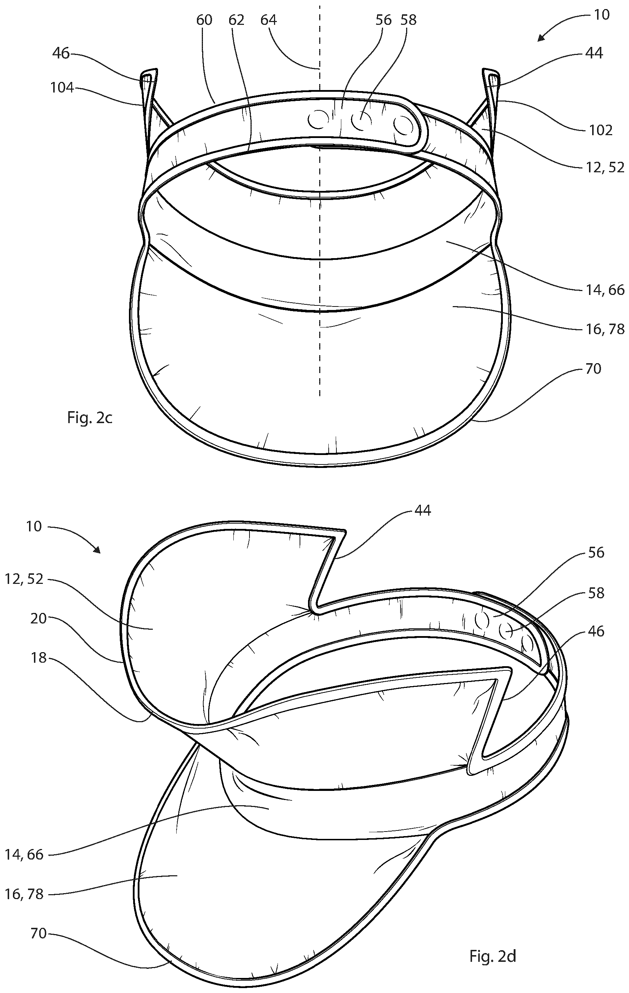

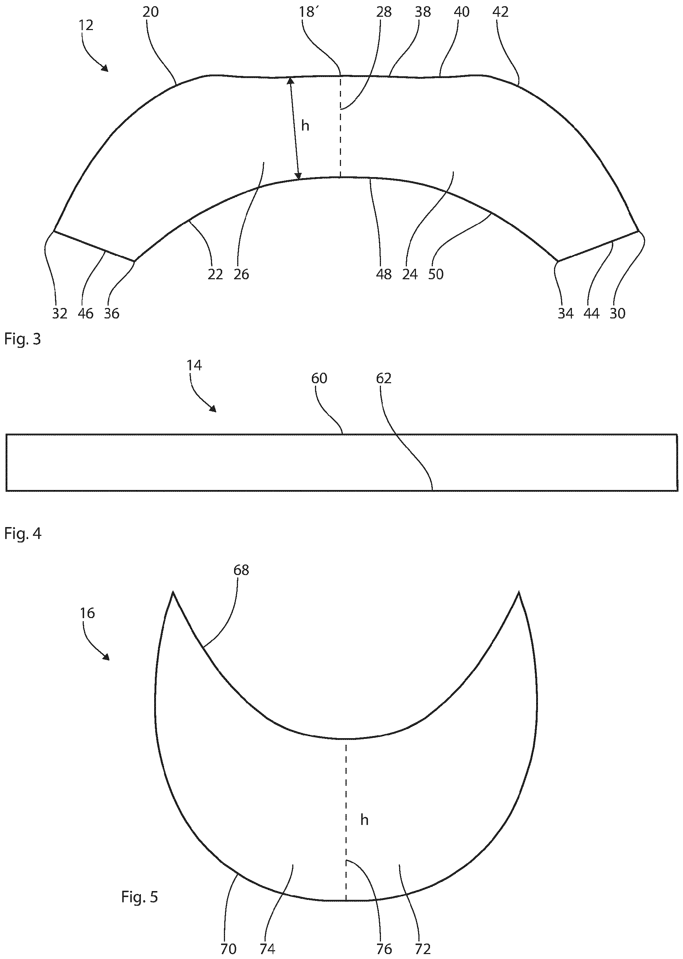

An embodiment of a wearable radiation-protective device 10 positioned at the neck of a user is shown in **Figs. 1a and 1b****.** The radiation-protective device 10 is shown in its intended position relative to the user. The user is standing upright on a horizontal surface with the face facing straight forward. Details of the radiation-protective device 10 are shown in **Figs. 2a to 2d** with the radiation-protective device 10 in the corresponding intended position during use. The radiation-protective device has a head shield 12, a neck shield 14, and a chest shield 16 that are connected. Each of the head shield 12, neck shield 14, and chest shield 16 has a curved shape in the intended position, and each of the head shield 12, neck shield 14, and chest shield 16 can be flattened to a planar shape on a planar surface, as shown in **Figs. 3, 4, and 5****.**

The head shield 12, neck shield 14, and chest shield 16 are each of a single piece of the same X-ray attenuating material. In alternative embodiments, the different parts 12, 14, and 16 are composite structures of several overlapping pieces of an X-ray attenuating material that are joined together by construction stiches, or that are welded or glued together. In other embodiments, the parts 12, 14, and 16 are of different X-ray attenuating materials.

The X-ray attenuating material can attenuate X-ray radiation from a radiation source having a tube voltage in the range of 40-150 kV. The X-ray attenuating material has a lead equivalent of 0.35 mm Pb equivalence.

The X-ray attenuating material is a permeable fabric that comprises monofilaments arranged in a regular pattern and extending in a uniform direction, for example as described in WO2014163574A1. Each filament is solid composition that comprises a carrier substance of polyolefin and radiopaque substance of Antimony and Bismuth powder that are uniformly mixed. The fabric is a woven fabric with the filaments forming the weft of the cloth and with a warp of polyester. This way, each of the head shield 12, neck shield 14, and chest shield 16, is of a of sheet-like, pliable, and radiation attenuating material, the head shield 12 is arranged to conform to the chin of the user, the neck shield 14 is arranged to conform to the neck of the user, and the chest shield 16 is arranged to conform to the chest of the user, as shown in Figs 1a and 1b.

In alternative embodiments, the first material is an impermeable sheet and/or composed of several layers.

The radiation-protective device 10 has a head-part lining 52 that covers the head shield 12, a neck-part lining 66 that covers the neck shield 14, and a chest-part lining 78 that covers the chest shield 16. Each lining 52, 66, and 78 prevents direct access to the part 12, 14, and 16 it covers. The linings 52, 66, and 78 are of a fabric of polyester with water-proof breathable membrane.

The head shield 12 has an outer edge 20 and an inner edge 22, and the outer edge 20 extends upwards relative to the inner edge 22 in the intended position. The radiation-protective device 10 positions the complete inner edge 22 of the head shield 12 at the neck of the user in the intended position, which is apparent from the combined **Figs. 2a to 2d** **and** **3**. The head shield 12 extends upwards relative to and is fully supported by the neck shield 14 in the intended position. This way, the neck shield 14 is arranged to position the outer edge 20 of the head shield 12 at the chin of the user in the intended position.

The neck shield 14 as such has a curved shape, extends partly around the neck of the user, and conforms to the neck of the user in the intended position, as shown in **Figs. 1a and 1b**. radiation-protective device 10 has a neck-part extension 56 that is connected to and extends from the neck shield 14. The neck-part extension 56 is formed as an extension of the head-part lining 52 and has an overlap in the intended position at the back of the neck of the user, see for example **Figs. 1a and 1b****.** The neck-part extension 56 has a lock 58 at the overlap in the form of cooperating magnets that releasably lock the neck shield 14 around the neck of the user in the intended position. The combined neck shield 14 and neck-part extension 56 has a length of 65 cm.

The head shield 12 and the neck shield 14 overlap along the complete inner edge 22 by about 5 mm, and the head shield 12 is connected to the neck shield 14 at the inner edge 22 by a construction stich. In alternative embodiments, the head shield 12 is welded or glued to the neck shield 14. In its planar shape, the neck shield 14 part is elongated and has a straight first upper edge 60 and a straight first lower edge 62 that are parallel, as shown in **Fig. 4****.**

The chest shield 16 has a curved shape and covers an upper part of the chest at the front of the user in the intended position, as shown in **Figs. 1a and 1b****.** The chest shield 16 extends downwards from and is fully supported by the neck shield 14 in the intended position. The neck shield 14 is connected to the chest shield 16 at the first lower edge 62 of the neck shield 14 by a construction stich. The chest shield 16 has a concave second upper edge 68 and a convex second lower edge 70 that both outlines a smooth curve in the planar shape, as shown in **Fig. 5****.** The complete second upper edge 68 of the chest shield 16 is located at the first lower edge 62 of the neck shield 14, which is apparent from combined **Figs 2a to 2d** **and** **5**. The chest shield 16 and the neck shield 14 overlap by about 5 mm and are connected by a construction stich along the complete second upper edge 68 of the chest shield 16. In alternative embodiments, the chest shield 16 is welded or glued to the neck shield 14.

The head shield 12 has a forward end 18 in the intended position relative to the user, see for example **Figs. 1a and 1b****.** The corresponding forward end 18' in the planar shape is shown in **Fig. 3****.** The head shield has a right first side and an opposite left second side relative to the forward end in the intended position of the radiation-protective device. The head shield 12 has a first head-part section 24 and a second head-part section 26 that are divided by a first central line 28 that extends normal to the inner edge 22 and through the corresponding forward end 18' in the planar shape of the head shield 12. The first head-part section 24 is on the first side and the second head-part section is on the second side.

The outer edge 20 of the head shield 12 has a first end 30 located on the first head-part section 24 and a second end 32 located on the second head-part section 26. Similarly, the inner edge 22 of the head shield 12 has a first end 34 located on the first head-part section 24 and a second end 36 located on the second head-part section 26. The uniform direction of the filaments of the material of the head shield 12 are aligned with the first central line 28 in the planar shape.

The head shield 12 is symmetric in the planar shape of the head shield 12 with the first head-part section 24 and the second head-part section 26 being congruent and having a reflection symmetry relative to the first central line 28, as shown in **Fig 3****.**

The head shield 12 has a straight first side edge 44 that interconnects the first end 30 of the outer edge 20 and the first end 34 of the inner edge 22. Similarly, the head shield 12 has a straight second side edge 46 that interconnects the second end 32 of the outer edge 20 and the second end 36 of the inner edge 22. The uniform direction of the filaments in the material of the neck shield 14 is transverse to the first upper edge 60 and the first lower edge 62 of the neck shield 14.

In the planar form shown in **Fig. 3****,** the outer edge 20 outlines an outer smooth curve and the inner edge 22 outlines an inner smooth curve. The outer edge 20 of the head shield 12 has a convex first outer-edge section 38 that extends from the first central line 28, a concave second outer-edge section 40 that extends from the first outer-edge section 38, and a convex third outer-edge section 42 that extends from the second outer-edge section 40 to the first end 34 of the outer edge 20.

The radiation protective device 10 positions the outer edge 20 on the first head-part section 24 on a first side of the neck of the user and the outer edge 20 on the second head-part section 26 on an opposite second side of the neck of the user, as shown in **Fig. 1b**. The outer edge 20 is located at neck of the user at the first side and at the second side of the neck. The complete inner edge 22 of the head shield 12 is located at the neck and first upper edge 60 of the neck shield 14.

The chest shield 16 has a first chest-part section 72 and a second chest-part section 74 that are divided by a second central line 76 that extends normal to the second upper edge 68 of the chest shield 16. The first chest-part section 72 and the second chest-part section 74 are congruent and have a reflection symmetry relative to the second central line 76 in the planar shape of the chest shield 16, as shown in **Fig. 5****.**

The head shield 12 has a height h in the planar shape of the head shield 12 between the inner edge 22 and the outer edge 20 and normal to inner edge 22, see **Fig. 3****.** The height h at the forward end 18 and along the first central line 28 is such that the head shield 12 and the neck shield 14 jointly positions the outer edge 20 at the forward end 18 at the level of and in front of the chin in the intended position, as shown in **Figs. 1a and 1b**.

The inner edge 22 has a first central-edge section 48 that extends from the first central line 28 to a point between the first central line 28 and the first end 34 of the inner edge 22. The first central-edge section 48 extends to about 5 cm along the inner edge 22 from the first central line 28. The height h across the first central-edge section 48 is less than 1.1 times the height at the first central line 28. The inner edge 22 has a first peripheral-edge section 50 that extends from the first central-edge section 48 to about 14 cm along the inner edge 22 from the first central line 28. The height h across the first peripheral-edge section 50 is greater than 1.1 times the height at the first central line 28. The inner edge 22 has a corresponding second central-edge section (not indicated) and second peripheral-edge section (not indicated) between the first central line 28 and the second end 36 of the inner edge 22.

The inner edge 22 on the first head-part section 24 has an arc length of about 17.5 cm and the outer edge 20 on the first head-part section 24 has an arc length of about 25 cm. This means that the outer edge 20 is about 1.4 times longer than the inner edge 22 on the first head-part section 24. The first side edge 44 has a length of about 6 cm, which means that it is shorter than the height at the first central line 28 by a factor of about 0.9.

The elongated neck shield 14 has a length along the first upper edge 60 and the first lower edge 62 of about 46 cm. The neck shield 14 has a width of about 4 cm transverse to the first upper edge 60 and the first lower edge 62.

The chest shield 16 has a height h in the planar shape of the chest shield 16 between the second upper edge 68 and the second lower edge 70 of the chest shield 16 and normal to second upper edge 68 of the chest shield 16, see **Fig. 5****.** The height along the second central line 76 is about 11 cm.

The head shield 12 has a first external angle α to a horizontal plane of about 30° in the intended position, as can be seen in **Fig. 2a****.** The head shield 12 has a second external angle β to a horizontal plane of about 85° at the first side and the second side of the neck in the intended position, as can be seen in **Fig. 2b****.** The first side edge 44 and the second side edge 46 of the head shield 12 has a third external angle γ to a horizontal plane of about 85° in the intended position, as can be seen in **Fig. 2a****.**

The neck shield 14 outlines a cylindrical geometry with a cylinder axis 64 that has an angle δ of about 65° to the horizontal plane in the intended position, as shown in **Fig. 2a****.** The head shield 12 has a fourth external angle ε to neck shield 14 at the forward end 18 that is about 145°. The shape of the head part 12 and the head shield 12conforms to the shape of the neck shield 14 on the first side and the second side of the neck of the user in the intended position, as shown in **Fig. 1b**. The head shield 12 has a fifth external angle ζ to the neck shield 14 of about 175° at the first side and second side of the neck, as shown in **Fig. 2b****.** The first side edge 44 and the second side edge 46 of the head shield 12 has a sixth external angle η to the first upper edge 60 of the neck shield 14 that is about 60°, as can be seen in **Fig. 2a****.**

The head shield 12 has an outer side surface 98 and an inner side surface 100. The outer side surface 98 faces away from the user and the inner side surface 100 faces towards the user in the intended position of the radiation-protective device 10. The head-part lining 52 is located at both the inner side surface 100 and the outer side surface 98 of the head shield 12. The head shield 12 has a first side 102 and an opposite second side 104 relative to the forward end 18 in the intended position of the radiation-protective device 10, see **Fig. 2b** **and** **2c****.**

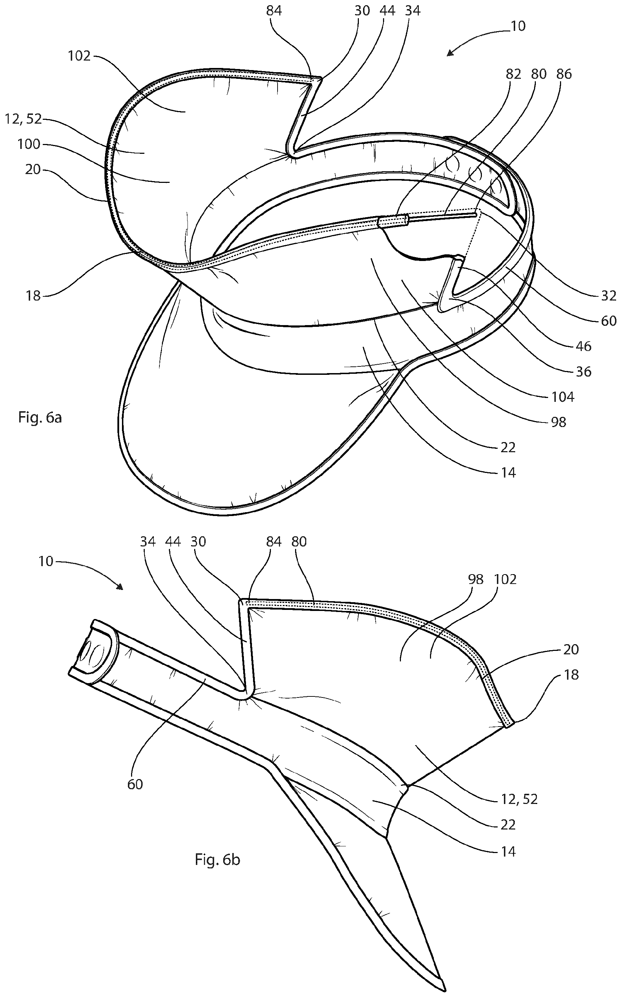

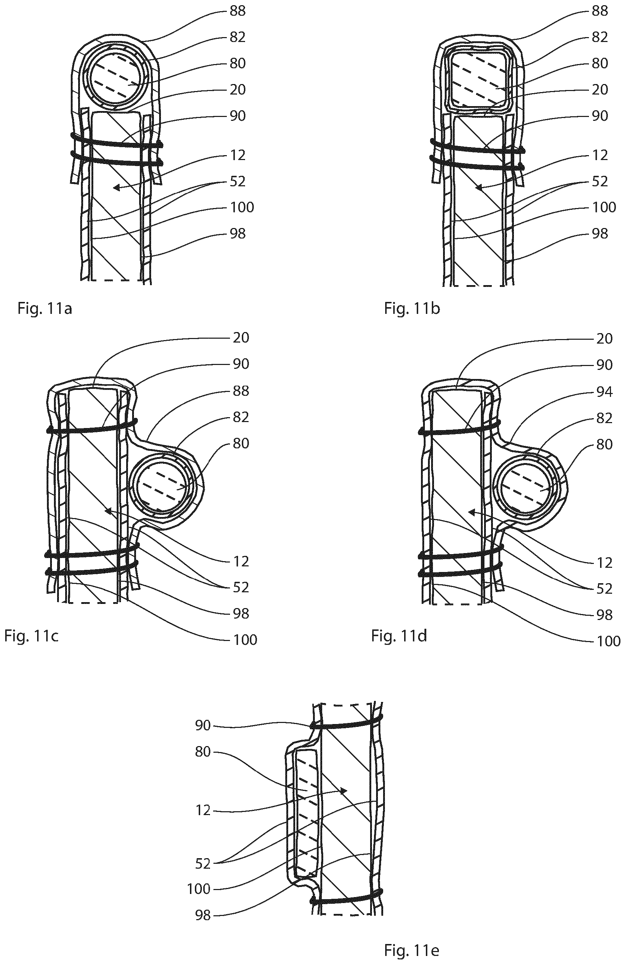

As shown in **Figs. 6a to 6b****,** the radiation-protective device 10 has an elongated support structure 80 in the form of a solid-core steel wire with a circular cross section. This means that the elongated support structure 80 is arranged to deform at a load transverse to the elongated support structure 80, and that it is plastically deformable by hand. As shown in **Fig. 11a****,** the radiation-protective device 10 further has a cover 82 in the form of a sleeve of silicone rubber that encloses and is detached the complete support structure 80 and elastically deforms at a deformation of the support structure 80.

The support structure 80 is located at and extends along the complete outer edge 20 of the head shield 12. The first end 84 of the support structure 80 is located at the outer edge 20 and at the first end 30 of the outer edge 20 of the head shield 12, the second end 86 of the support structure 80 is located at the outer edge 20 and at the second end 32 of the outer edge 20 of the head shield 12, and the support structure 80 extends across the forward end 18 of the head shield 12.

In an alternative embodiment, the support structure 80 extends only along a part of the outer edge 20. The first end 84 of the support structure 80 is then located on the first side 102 of the head shield 12 between the first end 30 of the outer edge 20 and the forward end 18, but closer to the first end 30 of the outer edge 20 than to the forward end 18. The second end 86 of the support structure 80 is arranged in a corresponding manner on the second side 104 of the head shield 12. In another embodiment, the first end 84 and the second end 86 of the support structure 80 are both located on the first side 102 of the head shield 12 and the support structure 80 does not extend across the forward end 18 of the head shield 12.

The support structure 80 is centred between the outer side surface 98 and the inner side surface 100, as shown in **Fig. 11a****,** and the cover 82 spaces the support structure 80 apart from the adjacent parts of the radiation-protective device 10.

The radiation-protective device 10 has a bias tape 88 in the form of a fabric tape that covers and folds over the outer edge 20, first side edge 44, and second side edge 46 of the head shield 12. The bias tape 88 also covers and folds over parts of the first upper edge 60 of the neck shield 14. The bias tape 88 is composed of several bias tape sections (not shown) and is attached to the head shield 12 and the neck shield 14 by sewn seams 90 at the respective edges. The bias tape 88 is located at and aligned with the support structure 80. It has opposite longitudinal sides that are located on the same side of the support structure 80, and it covers and folds over the complete support structure 80. The seams 90 are disjoint from the support structure 80 and extend along the support structure 80. This way, the bias tape 88 and the seams 90 jointly attach the support structure 80 to the head shield 12, and the support structure 80 is arranged to bias the head shield 12 to have a desired shape in the intended position. Further, a load can be applied to the support structure 80 via the head shield 12, the support cover 82, and the bias tape 88.

In an alternative embodiment shown in **Fig. 11b****,** the support structure 80 has a solid-core steel wire with a square cross section. In yet another embodiment shown in **Fig. 11c****,** the support structure 80 located on the outer side surface 98 of the head shield 12, and the bias tape 88 is attached to the head shield 12 by sewn seams 90 on both sides of the support structure 80. In yet another embodiment shown in **Fig. 11d****,** there is no bias tape 88 at the outer edge 20 of the head shield 12. Instead, the head-part lining 52 has a fold 94 that covers the outer edge 20 of the head shield 12 and the support structure 80. The head-part lining 52 is attached to the head shield 12 by sewn seams 90 on both sides of the support structure 80. This way, the head-part lining 52 and the seams 90 jointly attach the support structure 80 to the head shield 12.

In the embodiment of **Figs. 1a to 6****,** the wearable radiation-protective device 10 is positioned with the complete inner edge 22 of the head shield 12 at the neck of the user in the intended position. In an alternative embodiment, the wearable radiation-protective device 10 has no neck shield 14 and is positioned at the chest of the user and. The complete inner edge 22 of the head shield 12 is then positioned at the chest of the user in the intended position and connected directly to the chest shield 16 or another garment. In this embodiment, the head shield is spaced apart from the neck and the head of the user.

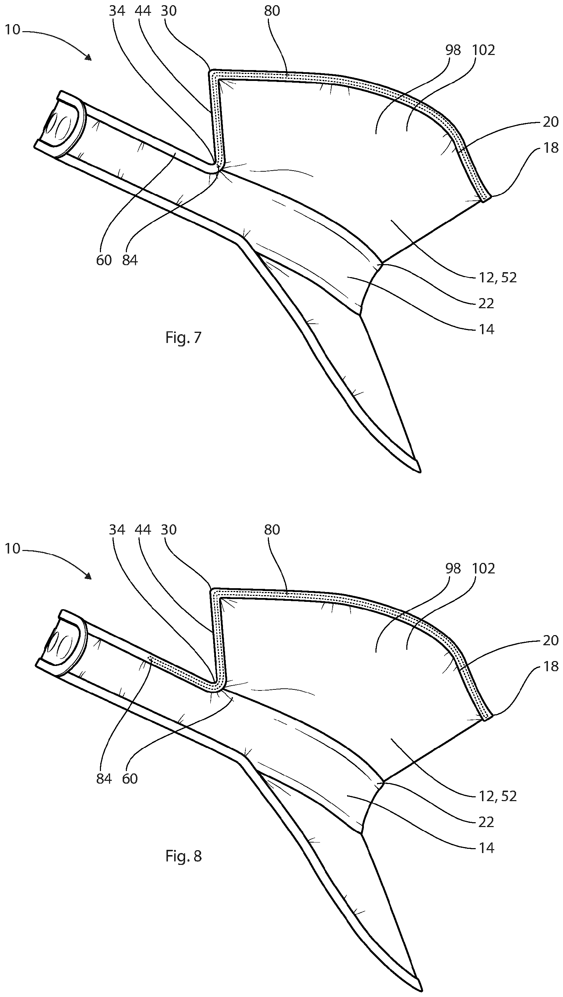

Another embodiment of a radiation-protective device 10 is shown in **Fig. 7****.** It differs from the embodiment of **Figs. 1a to 6** in that the support structure 80 is located at and extends along the complete outer edge 20, the first side edge 44, and the second side edge 46 of the head shield 12. The first end 84 of the support structure 80 is located at the first side edge 44 and at the first end 34 of the inner edge 22 of the head shield 12, the second end 86 of the support structure 80 is located at the second side edge 46 and at the second end 36 of the inner edge 22 of the head shield 12. This means that the first end 84 of the support structure 80 are located between the first end 30 of the outer edge 20 and the first end 34 of the inner edge 22, and that the second end 86 of the support structure 80 is located between the second end 32 of the outer edge 20 and the second end 36 of the inner edge 22. The support structure 80 extends across the connection between the outer edge 20 and the first side edge 44 and across the connection between the outer edge 20 and the second side edge 46, and it is bent at the connections.

In another embodiment, the support structure 80 extends only along a part of the first side edge 44. The first end 84 of the support structure 80 is then located between the first end 30 of the outer edge 20 and the first end 34 of the inner edge 22, but closer to the first end 34 of the inner edge 22 than to the first end 30 of the outer edge 20.

Another embodiment of a radiation-protective device 10 is shown in **Fig. 8****.** It differs from the embodiment of **Figs. 1a to 6** in that the support structure 80 is located at and extends along the complete outer edge 20, the complete first side edge 44, and the complete second side edge 46 of the head shield 12, and along parts of the first upper edge 60 of the neck shield 14. The first end 84 of the support structure 80 is located at the first upper edge 60 of the neck shield 14 on the first side 102 of the neck shield 14, and the second end 86 of the support structure 80 is located at the first upper edge 60 of the neck shield 14 on the opposite second side 104 of the head shield 12. At the first upper edge 60, the support structure 80 extends away from the first end 34 and the second end 36 of the inner edge 22 rearwards relative to the front end of the radiation-protective device 10. The support structure 80 extends across the connection between the outer edge 20 and the first side edge 44 of the head shield 12, across the connection between the outer edge 20 and the second side edge 46 of the head shield 12, across the connection between the first side edge 44 of the head shield 12 and the first upper edge 60 of the neck shield 14, and across the connection between the second side edge 46 of the head shield 12 and the first upper edge 60 of the neck shield 14. The support structure 80 is bent at the connections.

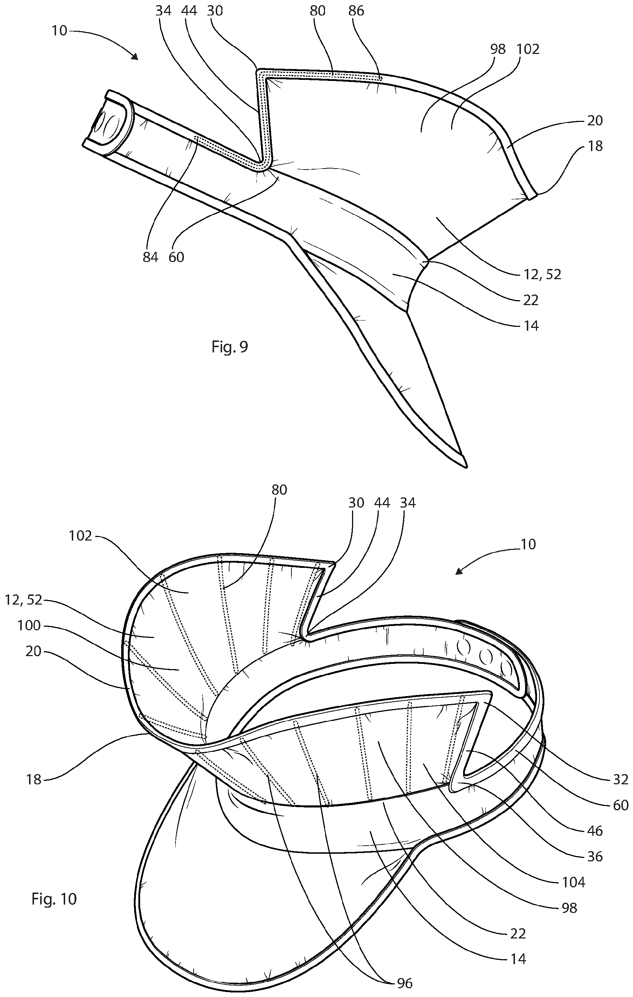

Another embodiment of a radiation-protective device 10 is shown in **Fig. 9****.** It differs from the embodiment of **Fig. 8** in that the support structure 80 extends only along a part of the outer edge 20 and not along the second side edge 46. The second end 86 of the support structure 80 is then located between the first end 30 of the outer edge 20 and the forward end 18, and closer to the first end 30 of the outer edge 20 than to the forward end 18. This means that the first end 84 and the second end 86 of the support structure 80 are located on the same first side 102 of the head shield 12.

In an alternative embodiment, the support structure 80 does not extend along the outer edge 20 and the second side edge 46. The second end 86 of the support structure 80 is then located on the first side edge 44 and at the first end 30 of the outer edge 20.

Another embodiment of a radiation-protective device 10 is shown in **Fig. 10**. It differs from the embodiment of **Figs. 1a to 6** in that the support structure 80 extends from the first peripheral-edge section of the inner edge 22 to the outer edge 20 of the head shield 12 transversely to the inner edge 22. The support structure 80 is fixed to the head shield 12 at and between the inner edge 22 and the outer edge 20 of the head shield 12.

The support structure 80 is located at the inner side surface 100 of the head shield 12, as shown in **Fig. 11e****.** In an alternative embodiment, it is located at the outer side surface 98 of the head shield 12. The support structure 80 is spaced apart from the first end 30 and the second end 32 of the outer edge 20 and from the first end 34 and the second end 36 of the inner edge 22. The support structure 80 is spaced apart from the forward end 18 of the radiation-protective device 10 and located closer to the first end 30 of the outer edge 20 and the first end 34 of the inner edge 22 than to the forward end 18 of the head shield 12.

The embodiment shown in **Fig. 10** further differs in that it has no cover 82 that encloses the support structure 80, and that the support structure 80 is a band with a flattened and rectangular cross section. The head-part lining 52 is attached to the head shield 12 on both sides of the support structure 80 by sewn seams 90 that are disjoint from and extend along the support structure 80, as shown in **Fig. 11e****.** This way, the head-part lining 52 and the seams 90 jointly attaches the support structure 80 to the head shield 12. The radiation-protective device 10 further has additional support structures 96 sharing the features of the support structure 80 and arranged relative to the head shield 12 as shown in **Fig. 10**.

### Item list

10 radiation-protective device
12 head shield
14 neck shield
16 chest shield
18 forward end
20 outer edge of head shield
22 inner edge of head shield
24 first head-part section
26 second head-part section
28 first central line
30 first end of outer edge
32 second end of outer edge
34 first end of inner edge
36 second end of inner edge
38 first outer-edge section
40 second outer-edge section
42 third outer-edge section
44 first side edge
46 second side edge
48 first central-edge section
50 first peripheral-edge section
52 head-part lining
56 neck-part extension
58 lock
60 first upper edge of neck shield
62 first lower edge of neck shield
64 cylinder axis
66 neck-part lining
68 second upper edge of chest shield
70 second lower edge of chest shield
72 first chest-part section
74 second chest-part section
76 second central line
78 chest-part lining
80 support structure
82 cover
84 first end of support structure
86 second end of support structure
88 bias tape
90 seams
94 fold
96 additional support structures
98 outer side surface
100 inner side surface
102 first side
104 second side

## Claims

1. A wearable radiation-protective device (10), that has an intended position at the neck of a user, the radiation-protective device (10) comprises:
- a head shield (12) and
- a support structure (80), wherein
the head shield (12) is a of sheet-like, pliable, and radiation attenuating first material, the head shield (12) has an outer edge (20) and an inner edge (22), the radiation-protective device (10) is arranged to position the inner edge (22) at the neck of the user in the intended position, the head shield (12) extends upwards from the inner edge (22) to the outer edge (20) in the intended position, the support structure (80) is attached to the head shield (12), the support structure (80) is deformable by hand, and the support structure (80) is arranged to bias the head shield (12) to have a desired shape in the intended position.

2. The wearable radiation-protective device (10) according to claim 1, wherein the support structure (80) is plastically deformable.

3. The wearable radiation-protective device (10) according to claim 1 or 2, wherein the support structure (80) is elongated.

4. The wearable radiation-protective device (10) according to claim 3, wherein the radiation-protective device (10) comprises:
- a cover (82), wherein
the cover (82) encloses at least parts of the support structure (80), and the cover (82) is elastically deformable at a deformation of the support structure (80).

5. The wearable radiation-protective device (10) according to claim 4, wherein the cover (82) is a sleeve, and the support structure (80) is detached from the sleeve.

6. The wearable radiation-protective device (10) according to any of the claims 3 to 5, wherein the support structure (80) is attached to the head shield (12) at the outer edge (20), and the support structure (80) extends along the outer edge (20).

7. The wearable radiation-protective device (10) according to claim 6, wherein the radiation-protective device (10) further comprises:
- a bias tape (88) that is attached to the head shield (12) at the outer edge (20), the bias tape (88) folds over the outer edge (20) and the support structure (80), and the bias tape (88) attaches the support structure (80) to the head shield (12).

8. The wearable radiation-protective device (10) according to any of the claims 3 to 7, wherein the head shield (12) has a first side edge (44) that interconnects the outer edge (20) and the inner edge (22), and the support structure (80) is attached to the head shield (12) at the first side edge (44), and the support structure (80) extends along the first side edge (44).

9. The wearable radiation-protective device (10) according to any of the claims 3 to 8, wherein the radiation-protective device (10) further comprises:
- a neck shield (14), wherein
the neck shield (14) is a of sheet-like, pliable, and radiation attenuating second material, the neck shield (14) is adapted to extend at least partly around the neck of the user in the intended position, the head shield (12) is connected to the neck shield (14), the neck shield (14) has a first upper edge (60), the inner edge (22) of the head shield (12) is located at the first upper edge (60), and the support structure (80) is attached to the neck shield (14) at the first upper edge (60), and the support structure (80) extends along the first upper edge (60).

10. The wearable radiation-protective device (10) according to any of the claims 3 to 9, wherein the head shield (12) has a first side (102) and an opposite second side (104) relative to the forward end in the intended position of the radiation-protective device (10), and the support structure (80) is located on the first side (102) and the second side (104) of the head shield (12).

11. The wearable radiation-protective device (10) according to any of the claims 3 to 9, wherein the head shield (12) has a first side (102) and an opposite second side (104) relative to the forward end in the intended position of the radiation-protective device (10), and the support structure (80) is located on the first side (102) of the head shield (12) and spaced apart from the second side (104) of the head shield (12).

12. The wearable radiation-protective device (10) according to any of the claims 3 to 5, wherein the head shield (12) has a first side edge (44) that interconnects the outer edge (20) and the inner edge (22), the support structure (80) extends between the inner edge (22) and the outer edge (20) of the head shield (12), and the support structure (80) is spaced apart from the first side edge (44).

13. The wearable radiation-protective device (10) according to any of the claims 3 to 12, wherein the radiation-protective device (10) further comprises:
- a head-part lining (52), wherein
the head-part lining (52) covers the head shield (12), the head-part lining (52) is of sheet-like and pliable first lining material, and the head-part lining (52) attaches the support structure (80) to the head shield (12).

14. Use of the wearable radiation-protective device (10) according to any of the claims 1 to 13, for protecting a person from radiation during a medical intervention comprising the use of radiation.
